# EUROPEAN PATENT APPLICATION

(11) **EP 1 013 295 A1**
(43) Date of publication of application: **28.06.2000**
(21) Application number: 98941833.0
(22) Date of filing: 11.09.1998
(51) Int. Cl.: A61M 1/34, A61M 5/162

(54) **LEUKOCYTE REMOVING FILTER UNIT AND METHOD OF USING THE SAME**

(30) Priority: 12.09.1997 JP 26792597
(71) Applicant: ASAHI MEDICAL Co., Ltd., Tokyo 101-8482 (JP)
(72) Inventor: YAJIMA, Kentaro, Minerubatsurusaki 602, Oita-shi, Oita 870-0105 (JP); OKA, Shinichiro, Oita-shi, Oita 870-0163 (JP)
(74) Representative: von Kreisler, Alek, Dipl.-Chem.
(86) International application number: JP9804101
(87) International publication number: WO9913925

(57) **Abstract**

A leukocyte-removing filter unit comprising at least the following components a) to e):
a) a puncture needle 1 having therein a first flow path and a second flow path which are independent of each other,
b) a first conduit 11 connected to the downstream end of the first flow path of the puncture needle,
c) a leukocyte-removing filter apparatus 2 comprising at least an upstream space connected to the downstream end of the first conduit, a filter medium and a downstream space,
d) a second conduit 12 connected to the downstream space of the leukocyte-removing filter apparatus, and
e) a bypass tube 15 one end of which is connected to the second flow path of the puncture needle 1 and the other end of which is connected to at least one of the downstream space in the leukocyte-removing filter apparatus 2 and the second conduit 12.

## Description

### TECHNICAL FIELD

The present invention relates to a leukocyte-removing filter unit used for removing leukocytes from blood and blood components, and a method for using the same.

### BACKGROUND ART

In recent years, so-called component transfusion comprising separating blood into individual components and transfusing only a blood component suitable for purpose of therapy have been generalized owing to the progress of medical science, in particular, immunology.

However, leukocytes contained as a contaminant in a blood component for transfusion at the time of transfusion are those of another person. Since there are a wide variety of blood types of leukocytes, it is difficult to carry out transfusion by completely matching a patient's leukocyte type, even when the ABO blood group of erythrocytes of the patient matches with that of a donor. Therefore, an antibody is produced in the patient's body against an antigen present on the surface of leukocytes transfused, and repeated transfusion causes antigen-antibody reaction between the anti-leukocyte antibody in the patient's body and leukocytes in transfused blood, so that side effects of transfusion, such as febrific reaction, hives, etc. are often caused.

Accordingly, leukocyte-removing filters for removing leukocytes contained in a blood component for transfusion have recently been developed. These leukocyte-removing filters can efficiently remove leukocytes in whole blood, red cell products, platelet products and the like, and hence prevent the side effects in patients who receive a transfusion. Therefore, they are eagerly welcomed by the patients.

Leukocyte-removing filter units are often used by connecting a blood tube to each of the upper part and lower part of the unit and include, for example, bed side type ones mainly for direct transfusion into a patient, and laboratory type ones for preparing a leukocyte-free blood product in a Blood Center.

In the bed side type ones, for example, a puncture needle for connection to a blood container (a blood product bag) containing blood is connected so as to be upstream to a leukocyte-removing filter apparatus through a blood tube and, for example, a drip chamber and a connecting adapter for connecting a transfusion needle are connected so as to be downstream to the leukocyte-removing filter apparatus through a blood tube.

In the laboratory type ones, for example, a puncture needle for connection to a blood container (a blood product bag) containing blood is connected so as to be upstream to a leukocyte-removing filter apparatus through a blood tube and, for example, a blood container (a blood product bag) for recovering the resulting blood product freed of leukocytes is connected so as to be downstream to the leukocyte-removing filter apparatus through a blood tube.

Such conventional leukocyte-removing filters, however, are filters obtained by using nonwoven fabric made of very fine fiber or a porous material having a small pore size, and hence have a very low gas permeability after the material therefor is once wetted. Therefore, the conventional leukocyte-removing filters are disadvantageous in that a blood product such as blood or a blood component, which is effective in treating a patient, remains in the leukocyte-removing filter unit unless a recovery procedure is carried out after completion of filtration. Therefore, the improvement of blood tubes is desired for efficient utilization of the blood product such as blood or blood component which remains in the leukocyte-removing filter unit.

As a method for recovering the blood product remaining in the leukocyte-removing filter unit, there is a method of allowing a physiological solution such as physiological saline to flow from portions upstream to a leukocyte-removing filter apparatus to replace the inner atmosphere of the leukocyte-removing filter unit with the physiological solution and thereby recovering the remaining blood product. However, in this method, contamination with bacteria can take place not only when a puncture needle is connected to a blood container containing a blood product to be filtered, but also when the puncture needle is connected to a bottle of physiological saline. Therefore, this method gives one more chance of contamination and requires troublesome operations from persons engaged in remedy.

JP-A-5-500820 and JP-A-6-063132 disclose methods comprising providing in a leukocyte-removing filter unit a sterite filter which permits entrance and exit of gas, and introducing atmospheric air into the leukocyte-removing filter unit to carry out the recovery. Employment of this method, however, is prohibited in some countries because the atmospheric air disinfected by the disinfection filter is very likely to be more contaminated than sterile air, and the absence of the quality problem in the disinfection filter, i.e., pinholes cannot be warranted, so that there is a possibility of contamination with bacteria. Moreover, it is impossible to recover a blood product remaining in portions upstream to the place of providing the disinfection filter and in a container which contains blood product to be filtered.

In addition, JP-A-7-194678 discloses a method in which when a blood product is introduced into a leukocyte-removing filter unit, sterile air previously sealed up inside the leukocyte-removing filter unit is reserved in a blood container (a blood product bag) provided for recovering blood product freed of leukocytes, or in a small container for air reservation provided in the leukocyte-removing filter unit for the purpose of reserving the sterile air, and blood product remaining in the leukocyte-removing filter unit is recovered by utilizing the reserved sterile air. This method, however, is disadvantageous as follow: the blood container (blood product bag) or the small container for gas reservation is absolutely necessary in order to reserve the sterile air, an increase in cost is unavoidable, and troublesome operations such as the transfer of the reserved sterile air to portions upstream to a filter apparatus are required of persons engaged in remedy.

The present invention is intended to provide a leukocyte-removing filter unit for treating blood or a blood component which makes it possible to treat blood or a blood component while efficiently recovering residual blood or blood component in the leukocyte-removing filter unit without contact of the residual blood or blood component with the air, by an aseptic and simple procedure, and requires only easy operations and a low cost.

### DISCLOSURE OF THE INVENTION

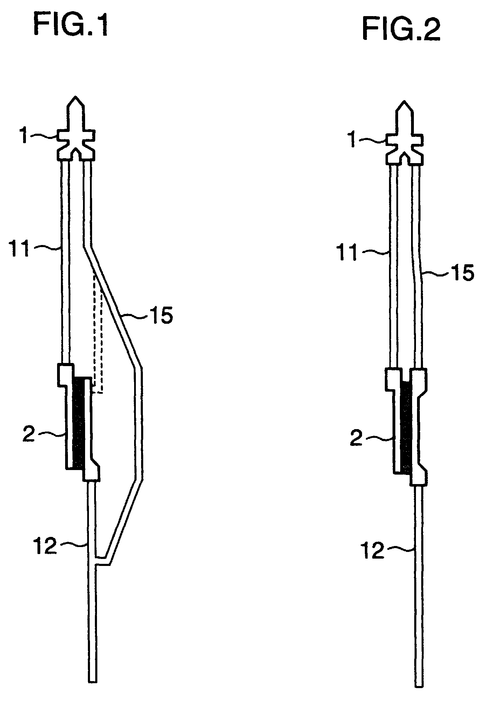

The present inventors earnestly investigated in order to solve the above problems, and consequently solved them by, as shown in the schematic view Fig. 1, using a puncture needle having therein two independent flow paths, i.e., a first flow path and a second flow path, in place of a conventional puncture needle for blood container, allowing a blood product to flow into a leukocyte-removing filter through the first flow path, providing a bypass linking one or two portions down-stream to the leukocyte-removing filter apparatus to the second flow path of the puncture needle, and returning sterile air in the leukocyte-removing filter unit to the blood-to-be-filtered side, whereby the present invention has been accomplished.

That is, one aspect of the present invention is directed to a leukocyte-removing filter unit comprising at least the following components a) to e) as shown in Fig. 1:
a) a puncture needle (1) having therein a first flow path and a second flow path which are independent of each other,
b) a first conduit (11) connected to the down-stream end of the first flow path of said puncture needle,
c) a leukocyte-removing filter apparatus (2) comprising at least an upstream space connected to the downstream end of said first conduit, a filter medium and a downstream space,
d) a second conduit (12) connected to the down-stream space of said leukocyte-removing filter apparatus, and
e) a bypass tube (15) one end of which is connected to the second flow path of said puncture needle (1) and the other end of which is connected to at least one of the downstream space in said leukocyte-removing filter apparatus (2) and said second conduit (12).

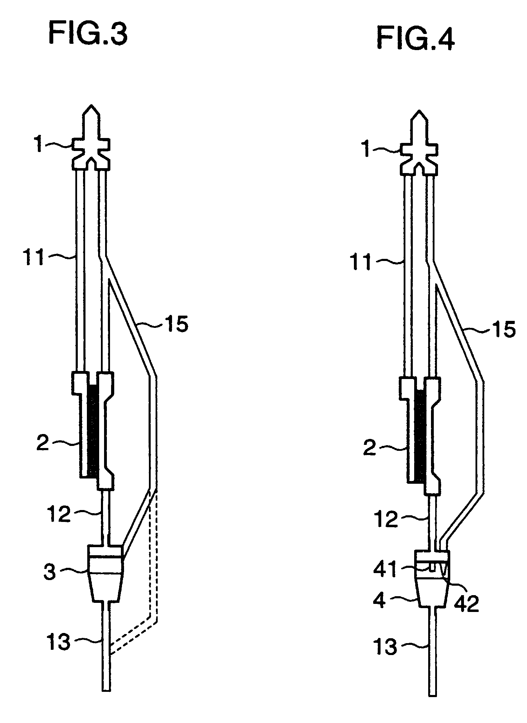

Another aspect of the present invention is directed to a leukocyte-removing filter unit comprising at least the following components a) to g) as shown in Fig. 3:
a) a puncture needle (1) having therein a first flow path and a second flow path which are independent of each other,
b) a first conduit (11) connected to the down-stream end of the first flow path of said puncture needle,
c) a leukocyte-removing filter apparatus (2) comprising at least an upstream space connected to the downstream end of said first conduit, a filter medium and a downstream space,
d) a second conduit (12) connected to the down-stream space of said leukocyte-removing filter apparatus,
e) a drip chamber (3) connected to the down-stream end of said second conduit,
f) a third conduit (13) connected to the down-stream end of said drip chamber, and
g) a bypass tube (15) one end of which is connected to the second flow path of said puncture needle (1) and the other end of which is connected to at least one of the downstream space in said leukocyte-removing filter apparatus (2), said drip chamber (3) and said third conduit (13).

Further another aspect of the present invention is directed to a leukocyte-removing filter unit comprising at least the following components a) to g) as shown in Fig. 4:
a) a puncture needle (1) having therein a first flow path and a second flow path which are independent of each other,
b) a first conduit (11) connected to a down-stream end of the first flow path of said puncture needle,
c) a leukocyte-removing filter apparatus (2) comprising at least an upstream space connected to a down-stream end of said first conduit, a filter medium and a downstream space,
d) a second conduit (12) connected to the down-stream end of said leukocyte-removing filter apparatus,
e) a drip chamber (4) which is equipped with a nozzle (41) that communicates with the upper space in the drip chamber, and a flow path (42) one end of which opens outside said drip chamber and the other end of which opens downstream of said nozzle (41) in said drip chamber, said drip chamber (4) being connected to the downstream end of the second conduit (12) via said nozzle,
f) a third conduit (13) connected to the down-stream outlet of said drip chamber, and
g) a bypass tube (15) one end of which is connected to the second flow path of said puncture needle (1) and the other ends of which are connected to the down-stream space in said leukocyte-removing filter apparatus (2) and the flow path (42), respectively.

Still another aspect of the present invention is directed to a leukocyte-removing filter unit having a gas-liquid separation filter (5) provided therein so as to block the above-mentioned bypass tube (15).

Still another aspect of the present invention is directed to a method for removing leukocytes from blood or a blood component by using the leukocyte-removing filter unit shown in Fig. 1, which comprises carrying out at least the following steps a) to e) in this order:
a) placing the puncture needle (1) in communication with a blood container containing blood to be filtered, so as not to permit the passage of liquid either in or out,
b) causing the blood in said blood container to flow down through the first flow path of the puncture needle (1) to the first conduit (11), the leukocyte-removing filter apparatus (2) and then the second conduit (12),
c) causing sterile air inside the leukocyte-removing filter unit to flow into and remain in said blood container through the bypass tube (15) simultaneously with the above step b),
d) discharging the blood remaining in the leukocyte-removing filter unit in the direction of the second conduit (12) by moving the sterile air in said blood container, through the first flow path of the puncture needle (1) to the first conduit (11) and then to the upstream space in the leukocyte-removing filter apparatus (2), after the whole of the blood in said blood container has flowed out, and/or
e) discharging the blood remaining in the leukocyte-removing filter unit through the second conduit (12) by moving the sterile air in said blood container, through the second flow path of the puncture needle (1) and the bypass tube (15) to at least one of the downstream space in the leukocyte-removing filter apparatus (2) and the second conduit (12).

Still another aspect of the present invention is directed to a method for removing leukocytes from blood or a blood component by using the leukocyte-removing filter unit shown in Fig. 3 which comprises carrying out at least the following steps a) to e) in this order:
a) placing the puncture needle (1) in communication with a blood container containing blood to be filtered, so as not to permit the passage of liquid either in or out,
b) causing the blood in said blood container to flow down through the first flow path of the puncture needle (1) to the first conduit (11), the leukocyte-removing filter apparatus (2), the second conduit (12) and then the drip chamber (3),
c) causing sterile air inside the leukocyte-removing filter unit to flow into and remain in said blood container through the bypass tube (15) simultaneously with the above step b),
d) discharging the blood remaining in the leukocyte-removing filter unit in the direction of the second conduit (12) by moving the sterile air in said blood container, through the first flow path of the puncture needle (1) to the first conduit (11) and then to the upstream space in the leukocyte-removing filter apparatus (2), after the whole of the blood in said blood container has flowed out, and/or
e) discharging the blood remaining in the leukocyte-removing filter unit through the third conduit (13) by moving the sterile air in said blood container, through the second flow path of the puncture needle (1) and the bypass tube (15) to at least one of the downstream space in the leukocyte-removing filter apparatus (2), the drip chamber (3) and the third conduit (13).

Still another aspect of the present invention is directed to a method for removing leukocytes from blood or a blood component by using the leukocyte-removing filter unit shown in Fig. 4 which comprises carrying out at least the following steps a) to e) in this order:
a) placing the puncture needle (1) in communication with a blood container containing blood to be filtered, so as not to permit the passage of liquid either in or out,
b) causing the blood in said blood container to flow down through the first flow path of the puncture needle (1) to the first conduit (11), the leukocyte-removing filter apparatus (2), the second conduit (12) and then the drip chamber (4),
c) causing sterile air inside the leukocyte-removing filter unit to flow into and remain in said blood container through the bypass tube (15) simultaneously with the above step b),
d) discharging the blood remaining in the leukocyte-removing filter unit in the direction of the second conduit (12) by moving the sterile air in said blood container, through the first flow path of the puncture needle (1) to the first conduit (11) and then the upstream space in the leukocyte-removing filter apparatus (2), after the whole of the blood in said blood container has flowed out, and/or
e) discharging the blood remaining in the leukocyte-removing filter unit through the third conduit (13) by moving the sterile air in said blood container, through the second flow path of the puncture needle (1) and the bypass tube (15) to the downstream space in the leukocyte-removing filter apparatus (2) and/or the drip chamber (4).

Still another aspect of the present invention is directed to a puncture needle which has a first flow path (101) having an opening (101a) at one end on the puncture portion (103) and another opening (101b) at the other end, and a second flow path (102) having an opening (102a) at one end on the puncture portion (103) and another opening (102b) at the other end, wherein said first flow path and said second flow path are independent of each other, said opening (101a) is in a position lower than said opening (102a), and the cross-sectional area of said opening (101a) is larger than that of said opening (102a).

### BRIEF DESCRIPTION OF THE DRAWINGS

Fig. 1 is a schematic diagram showing one example of basic structure of the leukocyte-removing filter unit of the present invention.
Fig. 2 is a schematic diagram showing another example of basic structure of the leukocyte-removing filter unit of the present invention.
Fig. 3 is a schematic diagram showing further another example of basic structure of the leukocyte-removing filter unit of the present invention.
Fig. 4 is a schematic diagram showing still another example of basic structure of the leukocyte-removing filter unit of the present invention.
Fig. 5 is a vertical cross-sectional view showing a preferable embodiment of the puncture needle of the present invention.
Fig. 6 is a schematic view showing one example of the leukocyte-removing filter unit of the present invention.
Fig. 7 is a schematic view showing another example of the leukocyte-removing filter unit of the present invention.
Fig. 8 is a schematic view showing further another example of the leukocyte-removing filter unit of the present invention.
Fig. 9 is a schematic view showing one example of conventional leukocyte-removing filter unit.
Fig. 10 is a schematic view showing another example of conventional leukocyte-removing filter unit.
Fig. 11 is a vertical cross-sectional view showing a preferable embodiment of the drip chamber according to the present invention.
Fig. 12 is a vertical cross-sectional view showing another preferable embodiment of the drip chamber according to the present invention.

### BEST MODE FOR CARRYING OUT THE INVENTION

The puncture needle used in the present invention has at least two independent flow paths, i.e., a first flow path and a second flow path, inside the needle, has a double structure for allowing the needle to communicate with a blood container (a blood product bag) containing blood or a blood component, so as not to permit the passage of liquid either in or out, and makes it possible to recover sterile air sealed up inside a leukocyte-removing filter unit into the blood container through a bypass tube and reserve the same therein, while introducing the blood product in the blood container into the leukocyte-removing filter unit.

As such a puncture needle, for example, plastics double needles and metallic double needles can be used.

In the two independent flow paths, the first flow path and second flow path of the puncture needle, it is preferable to choose properly the sizes, positions, shapes and the like of the openings of the puncture portion in the blood container in order to prevent the incorporation of the sterile air recovered through the second flow path, into blood flowing through the first flow path, and thereby prevent the formation of an air block by this sterile air in the leukocyte-removing filter apparatus.

When the puncture needle is allowed to communicate with the blood container containing blood or a blood component, so as not to permit the passage of liquid either in or out, it is preferable, for example, to locate a first flow path having a large opening which is suitable for introducing the blood from the blood container, in the lower part of the puncture portion in the blood container, and locate a second flow path having a small opening which is suitable for discharging the sterile air in the leukocyte-removing filter unit into the blood container without introducing the sterile air into the first flow path, in the upper part of the puncture portion in the blood container.

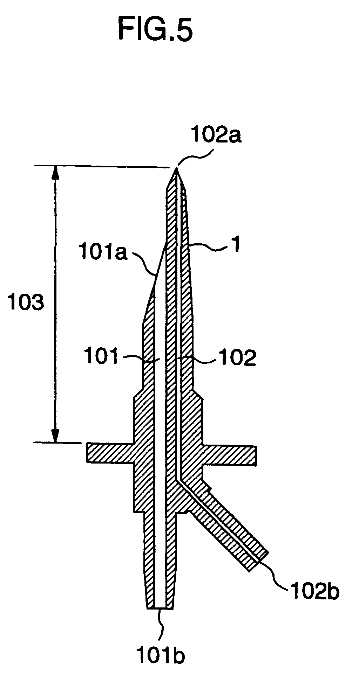

Fig. 5 is a vertical cross-sectional view showing a preferable embodiment of the puncture needle.

The puncture needle shown in Fig. 5 has a first flow path (101) and a second flow path (102) which are independent of each other. When the puncture needle is allowed to communicate with a blood container containing blood or a blood component which is to be filtered, so as not to permit the passage of liquid either in or out, the opening (101a) of the first flow path is located below the opening (102a) of the second flow path in the puncture portion (103) in the blood container, and the cross-sectional area of the opening (101a) of the first flow path is made larger than that of the opening (102a) of the second flow path. Thus, sterile air to be recovered into the blood container by its transfer in the direction of the opening (102b) of the second flow path and the opening (102a) of the second flow path through a bypass conduit connected to the second flow path so as not to permit the passage of liquid either in or out, can be prevented from being incorporated into the blood product flowing out of the blood container in the direction of the opening (101a) of the first flow path, the opening (101b) of the first flow path, and a first conduit connected to the first flow path so as not to permit the passage of liquid either in or out.

That is, it is preferable to choose properly the sizes, positions, shapes and the like of the openings of the puncture portion by taking, for example, the measures described below, in order to prevent the sterile air recovered through the second flow path from being incorporated into the blood product flowing through the first flow path, to form an air block in the leukocyte-removing filter apparatus: considering the viscosity and properties of the blood product, the openings of the first flow path and second flow path of the puncture needle are located at different heights in the axial direction of the puncture portion, and the cross-sectional area of the openings of the first flow path is made larger than that of the openings of the second flow path.

The first conduit (11), second conduit (12), third conduit (13) and bypass tube (15) referred to herein are hollow tubes for connecting the components so as not to permit the passage of liquid either in or out, and passing therethrough a gas and/or a blood product. As these tubes, flexible tubes are preferably used.

To these tubes, blocking devices such as clamps, roller clamps equipped with a flow regulator, communicating pieces (fragile connectors) and forceps, hydrophobic filters, safety valves such as check valves can be attached depending on purpose of use.

The leukocyte-removing filter apparatus (2) according to the present invention comprises at least an upstream space, a leukocyte-removing filter medium and a downstream space, and selectively captures only leukocytes, or leukocytes and platelets in a blood product but hardly captures other blood components. As the filter apparatus (2), there can be used conventional filters for removing leukocytes obtained by packing with a fibrous material, a porous material, a granular material or a combination thereof.

As the filter medium, either hydrophilic materials or hydrophobic materials can be used.

The words "upstream" and "downstream" or the words "above" and "below" which are used herein are defined on the basis of the direction of flow of a blood product. In the leukocyte-removing filter unit of the present invention, the blood product flows downward owing to a gravity head. Therefore, the term "portions upstream to the leukocyte-removing filter apparatus" means portions upstream to the filter medium of the leukocyte-removing filter apparatus, such as the puncture needle, the first conduit and the upstream space in the leukocyte-removing filter apparatus, and the term "portions downstream to the leukocyte-removing filter apparatus" means portions downstream to the filter medium of the leukocyte-removing filter apparatus, such as the downstream space in the leukocyte-removing filter apparatus, the second conduit, the drip chamber, the third conduit and a second reservoir.

As to the form of the filter medium, fibrous materials or porous materials are preferable because of their high leukocyte-capturing efficiency.

As to the form of the fibrous materials, although materials in the form of any of woven fabric, nonwoven fabric, cotton and a combination thereof can be used, materials in the form of nonwoven fabric are especially preferable from the viewpoint of blood passableness and leukocyte-removing efficiency.

As the above-mentioned leukocyte-removing filter apparatus, there are mentioned, for example, those obtained by packing nonwoven fabric, i.e. an assembly of fibrous material into a container as a main material for filter. The diameter of the fibers is approximately 0.3 µm to 20 µm. As a raw material for the fibers, synthetic fibers, semisynthetic fibers such as regenerated cellulose, natural fibers such as cotton, inorganic fibers, etc. are used.

Of these, the synthetic fibers such as fibers of poly(ethylene terephthalate)s, nylons, polypropylenes, polyacrylonitriles, etc. are preferably used.

The surface of the nonwoven fabric can be coated with a polymer substantially incapable of capturing platelets, in order to reduce the platelet-capturing efficiency and make possible the selective adsorption of only leukocytes. As the coating material, there can be used polymeric materials having basic nitrogen-containing functional groups, such as copolymers of diethylaminoethyl (meth)acrylate and hydroxyethyl (meth)acrylate.

The drip chamber (3 or 4) used in the present invention is a container having a space which permits reservation of gas and liquid, like generally known ones, and it is usually cylindrical. The drip chamber has such a structure that when a blood product is reserved in the drip chamber in a volume of approximately one-third to one-half the capacity of the drip chamber and then allowed to flow, the blood product drips from a nozzle for dripping provided on the top surface of the drip chamber. The drip chamber makes it possible to use the volume of the blood product dripped, as a measure for adjusting the transfusion rate (treatment rate) and prevent the introduction of air into the vein of a patient. The drip chamber is connected between the downstream end of the second conduit (12) and the upstream end of the third conduit (13) so as not to permit the passage of liquid either in or out.

In some cases, a flow path (42) different from the second conduit (12) and the third conduit (13) is connected to the drip chamber so as not to permit the passage of liquid either in or out. The flow path (42) is a conduit one end of which is open in the drip chamber and the other end of which is connected to the bypass tube (15) so as not to permit the passage of liquid either in or out. Particularly when the position of the opening of this conduit in the drip chamber is downstream to the above-mentioned nozzle for dripping and is such that a blood product can be reserved in the drip chamber in a volume of approximately one-third to one-half the capacity of the drip chamber, the following becomes possible: without turning the drip chamber upside down as often carried out, the blood product is reserved in the drip chamber in a volume of one-third to one-half the capacity of the drip chamber, and sterile air in the leukocyte-removing filter unit is discharged into the flow path (42) and the bypass tube (15) simultaneously with the transfer of the blood product to the flow path.

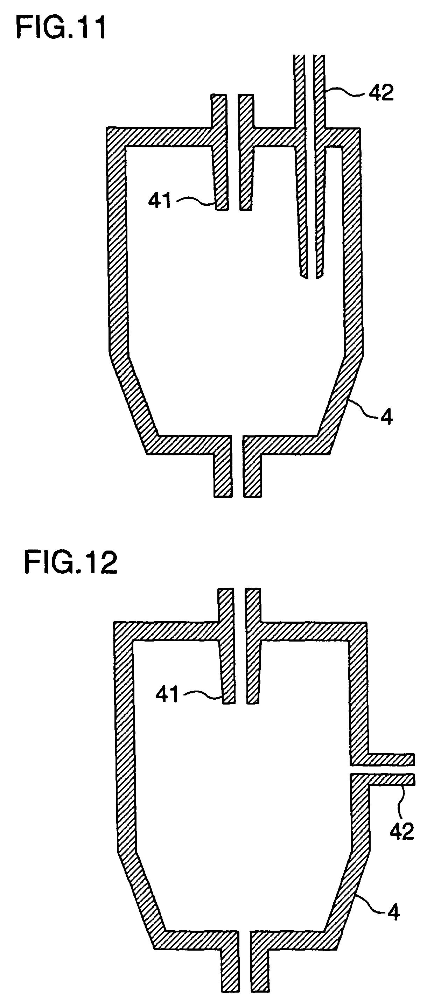

Fig. 11 is a vertical cross-sectional view showing a preferable embodiment of the drip chamber.

In the drip chamber (4) shown in Fig. 11, both a nozzle (41) and a flow path (42) are penetrated into the space in the chamber from the top of the drip chamber and have openings, respectively, in the space. The open tip of the flow path (42) is at a position below the open tip of the nozzle (41) at which a blood product is reserved in a volume about one-half the capacity of the drip chamber. Therefore, without turning the drip chamber upside down, the blood product dripped from the nozzle (41) is reserved in the cylinder of the drip chamber in a volume of about one-half the capacity of the drip chamber and it flows into the flow path (42) and then the bypass tube (15) to permit discharge of sterile air in the leukocyte-removing filter unit into the bypass tube (15).

Not only in Fig. 11, it is preferable to choose properly the sizes, relative positions, shapes and the like of the nozzle (41) and the flow path (42) in the drip chamber in order to prevent the following: in the nozzle (41) and the flow path (42), drips of a blood product from the nozzle (41) for dripping come into contact with the flow path (42) in the drip chamber to make impossible the determination of the volume of the blood product dripped, so that the adjustment of the transfusion rate or the treatment rate becomes impossible.

The gas-liquid separation filter(s) (5 and/or 17) used in the present invention is one which permits selective passage therethrough of gas and is impervious to blood products, such as hydrophobic filters. The gas-liquid separation filter(s) enables a blood product introduced into the leukocyte-removing filter unit, to stop when the blood product reaches the gas-liquid separation filter, and can prevent non-filtered blood product from being incorporated into leukocyte-free blood product downstream to the filter apparatus, from the second flow path of the puncture needle (1) through the bypass tube. The gas-liquid separation filter(s) is provided so as to block the bypass tube (15). Although a material for the hydrophobic filters is not particularly limited, polytetrafluoroethylenes (PTFE), poly(vinylidene difluoride)s (PVDF) and the like are preferably used.

In order to prevent their infection by bacteria, medical supplies such as leukocyte-removing filter units like that of the present invention are inserted into sterilization bags or the like and sterilized by γ-rays, ethylene oxide gas, autoclaving or the like, and at the same time, sterile air is sealed up inside the sterilization bags. The sterilization bags are bags or cases, which can be sterilized and whose interior can be kept sterile after the sterilization.

Next, the leukocyte-removing filter unit and the method for removing leukocytes from a blood product by the use of said leukocyte-removing filter unit of the present invention are explained below in further detail with reference to the drawings.

Fig. 1 is a schematic diagram showing one example of basic structure of the leukocyte-removing filter unit of the present invention, and shows that a bypass tube (15) is connected to at least one of the downstream space in a leukocyte-removing filter apparatus (2) and a second conduit (12).

Fig. 2 is a schematic diagram showing another example of basic structure of the leukocyte-removing filter unit of the present invention, and shows that a bypass tube (15) is connected to the downstream space in a leukocyte-removing filter apparatus (2).

Fig. 3 is a schematic diagram showing further another example of basic structure of the leukocyte-removing filter unit of the present invention, and shows that a bypass tube (15) is connected to the downstream space in a leukocyte-removing filter apparatus (2) and at least one of a drip chamber (3) and a third conduit (13).

Fig. 4 is a schematic diagram showing still another example of basic structure of the leukocyte-removing filter unit of the present invention, and shows that a bypass tube (15) is connected to the downstream space in a leukocyte-removing filter apparatus (2) and the flow path (42) of a drip chamber (4).

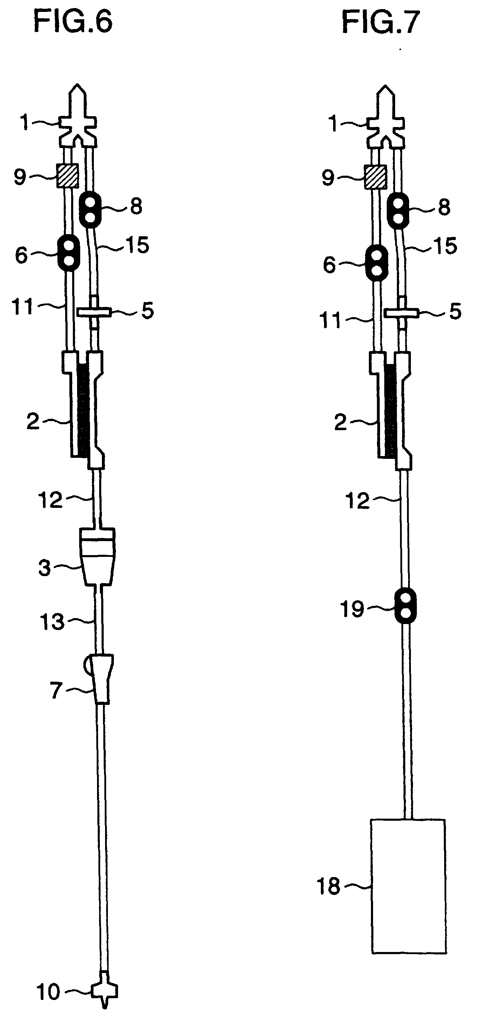

Fig. 6 is a schematic view showing one embodiment of the present invention.

In the embodiment shown in Fig. 6, blocking devices (6), (7) and (8) are attached to a first conduit (11), a third conduit (13) and a bypass tube (15), respectively, and can be opened or shut as required.

The blocking devices are devices for blocking the passage of gas and liquid, such as clamps, roller clamps equipped with a flow regulator, and forceps, and are preferably attached to the first conduit (11), a second conduit (12), the third conduit (13) and the bypass tube (15), depending on purpose of use. The blocking device (7) is preferably a roller clamp equipped with a flow regulator for the purpose of adjusting the rate of transfusion into a patient. As the blocking devices (6) and (8), any clamps can be used.

First, the blocking devices (6), (7) and (8) are shut at a state at which sterile air has been sealed up inside the leukocyte-removing filter unit, and a puncture needle (1) is allowed to communicate with a blood container containing a blood product to be filtered, so as not to permit the passage of liquid either in or out.

A gravity head of the blood product is attained in the leukocyte-removing filter unit either by opening the blocking devices (6) and (7) to introduce a small volume of the blood product into the first conduit (11) through the first flow path of the puncture needle (1), and then shutting the blocking device (7), or by attaching a first reservoir (9) made of a flexible material to the first conduit (11), grasping the first reservoir (9) and returning the same to the original state to introduce a small volume of the blood product into the first conduit (11) through the first flow path of the puncture needle (1), and then opening the blocking device (6).

The first reservoir is a container having a space which permits reservation of gas and liquid. It is preferable to reserve the blood product in the first reservoir in a volume of one-third to one-half the capacity of the reservoir, and connect the reservoir to the first conduit between the puncture needle (1) and a leukocyte-removing filter apparatus (2) so as not to permit the passage of liquid either in or out, in order to prevent the introduction of air into the leukocyte-removing filter apparatus and avoid blocking. It is also preferable to attach a filter for removing very small aggregates, to the first reservoir (9).

Then, the blocking device (8) is opened, upon which the blood product is transferred to the first conduit (11), the first reservoir (9), the leukocyte-removing filter apparatus (2) and then the bypass tube (15), and at the same time, the sterile air sealed up inside the leukocyte-removing filter unit is transferred to the first conduit (11), the first reservoir (9), the leukocyte-removing filter apparatus (2) and then the bypass tube (15), recovered into the blood container through the second flow path of the puncture needle (1) and reserved therein. Leukocytes in the blood product are removed by the passage of the blood product through the leukocyte-removing filter apparatus (2).

In this case, the blocking device (8) is shut immediately before the blood product introduced into the leukocyte-removing filter unit reaches the bypass (15). Alternatively, the leukocyte-removing filter unit is designed to stop the blood product when the blood product reaches a gas-liquid separation filter (5) provided in the bypass tube (15), and after the stop, the blocking device (8) is shut.

When the blocking device (7) is opened, the blood product is introduced into a drip chamber (3) through the second conduit (12). In this case, the drip chamber (3) is previously turned upside down and it is returned to its original state after the blood product is introduced into the drip chamber in a volume of one-third to one-half the capacity of the drip chamber. Then, the blood product is introduced into the third conduit (13).

A connecting adapter (10) is attached to the downstream (patient side) end of the third conduit (13). When the blood product reaches the connecting adapter (10), the blocking device (7) (a roller clamp equipped with a flow regulator) is shut and a transfusion needle is attached to the connecting adapter (10) to complete preparation for transfusion.

The connecting adapter (10) is an adapter for connecting a transfusion needle used for carrying out blood transfusion. As the connecting adapter (10), conventional ones such as a Luer-Locke type connector, expansion U-bend, etc. are preferably used. It is also possible to connect a transfusion needle to the connecting adapter (10) previously.

The blocking device (7) (roller clamp equipped with a flow regulator) is opened, the transfusion needle is filled with the blood product to the tip, the blocking device (7) is shut, and the transfusion needle is put into the vein of a patient. Then, the blocking device (7) is opened little by little and the transfusion rate is adjusted while counting drips from a nozzle for dripping in the drip chamber. Thus, the blood product freed of leukocytes is transfused into the patient.

After the whole of the blood product to be filtered in the blood container have flowed out into the leukocyte-removing filter unit, the sterile air reserved in the blood container is transferred through the first flow path of the puncture needle (1) to the first conduit (11), the first reservoir (9) and then the upstream space in the leukocyte-removing filter apparatus (2), whereby the residual blood product upstream to the leukocyte-removing filter apparatus in the leukocyte-removing filter unit can be transferred in the direction of the second conduit (12) to be recovered.

Further, the blocking device (8) is opened to transfer the sterile air reserved in the blood container, through the second flow path of the puncture needle (1) to the bypass tube (15) and then the down-stream space in the leukocyte-removing filter apparatus (2), whereby the residual blood product downstream to the leukocyte-removing filter apparatus in the leukocyte-removing filter unit can be transferred in the direction of the downstream (patient side) end of the third conduit (13) to the second conduit (12), the drip chamber (3) and then the third conduit (13) to be recovered.

The leukocyte-removing filter unit and the method for removing leukocytes from a blood product by the use of said leukocyte-removing filter unit of the present invention are explained below in still further detail with reference to the drawing.

Fig. 7 is a schematic view showing another embodiment of the present invention.

In the embodiment shown in Fig. 7, blocking devices (6), (19) and (8) are attached to a first conduit (11), a second conduit (12) and a bypass tube (15), respectively, and can be opened or shut as required. In addition, a second reservoir (18) is provided at the down-stream end of the second conduit (12).

The blocking devices are devices for blocking the passage of gas and liquid, such as clamps, roller clamps equipped with a flow regulator, and forceps, and are preferably attached to the first conduit (11), the second conduit (12) and the bypass tube (15), depending on purpose of use. As the blocking devices (6), (8) and (19), any clamps can be used.

The second reservoir is a flexible container having a space which permits reservation of gas and liquid, such as a blood container, and is intended for reserving a leukocyte-free blood product obtained by filtration by means of a leukocyte-removing filter apparatus (2). The second reservoir is preferably connected to the downstream end of the second conduit (12) so as not to permit the passage of liquid either in or out, when the leukocyte-removing filter unit is not used for direct administration of the leukocyte-free blood product to a patient.

First, the blocking devices (6), (8) and (19) are shut at a state at which sterile air has been sealed up inside the leukocyte-removing filter unit, and a puncture needle (1) is allowed to communicate with a blood container containing a blood product to be filtered, so as not to permit the passage of liquid either in or out.

A gravity head of the blood product is attained in the leukocyte-removing filter unit either by opening the blocking devices (6) and (19) to introduce a small volume of the blood product into the first conduit (11) through the first flow path of the puncture needle (1), and then shutting the blocking device (19), or by attaching a first reservoir (9) made of a flexible material to the first conduit (11), grasping the first reservoir (9) and returning the same to the original state to introduce a small volume of the blood product into the first conduit (11) through the first flow path of the puncture needle (1), and then opening the blocking device (6).

The first reservoir is a container having a space which permits reservation of gas and liquid. It is preferable to reserve the blood product in the first reservoir in a volume of one-third to one-half the capacity of the reservoir, and connect the reservoir to the first conduit between the puncture needle (1) and the leukocyte-removing filter apparatus (2) so as not to permit the passage of liquid either in or out, in order to prevent the introduction of air into the leukocyte-removing filter apparatus and avoid blocking. It is also preferable to attach a filter for removing very small aggregates, to the first reservoir (9).

Then, the blocking device (8) is opened, upon which the blood product is transferred to the first conduit (11), the first reservoir (9), the leukocyte-removing filter apparatus (2) and then the bypass tube (15), and at the same time, the sterile air sealed up inside the leukocyte-removing filter unit is transferred to the first conduit (11), the first reservoir (9), the leukocyte-removing filter apparatus (2) and then the bypass tube (15), recovered into the blood container through the second flow path of the puncture needle (1) and reserved therein. Leukocytes in the blood product are removed by the passage of the blood product through the leukocyte-removing filter apparatus (2).

In this case, the blocking device (8) is shut immediately before the blood product introduced into the leukocyte-removing filter unit reaches the bypass (15). Alternatively, the leukocyte-removing filter unit is designed to stop the blood product when the blood product reaches a gas-liquid separation filter (5) provided in the bypass tube (15), and after the stop, the blocking device (8) is shut.

When the blocking device (19) is opened, the blood product is introduced into the second reservoir (18) through the second conduit (12). Thus, the blood product freed of leukocytes is reserved in the second reservoir (18).

After the whole of the blood product to be filtered in the blood container have flowed out into the leukocyte-removing filter unit, the sterile air reserved in the blood container is transferred through the first flow path of the puncture needle (1) to the first conduit (11), the first reservoir (9) and then the upstream space in the leukocyte-removing filter apparatus (2), whereby the residual blood product upstream to the leukocyte-removing filter apparatus in the leukocyte-removing filter unit can be transferred in the direction of the second conduit (12) to be recovered.

Further, the blocking device (8) is opened to transfer the sterile air reserved in the blood container, through the second flow path of the puncture needle (1) to the bypass tube (15) and then the down-stream space in the leukocyte-removing filter apparatus (2), whereby the residual blood product downstream to the leukocyte-removing filter apparatus in the leukocyte-removing filter unit can be transferred in the direction of the downstream end of the second conduit (12) to be recovered.

The leukocyte-removing filter unit and the method for removing leukocytes from a blood product by the use of said leukocyte-removing filter unit of the present invention are explained below in still further detail with reference to the drawing.

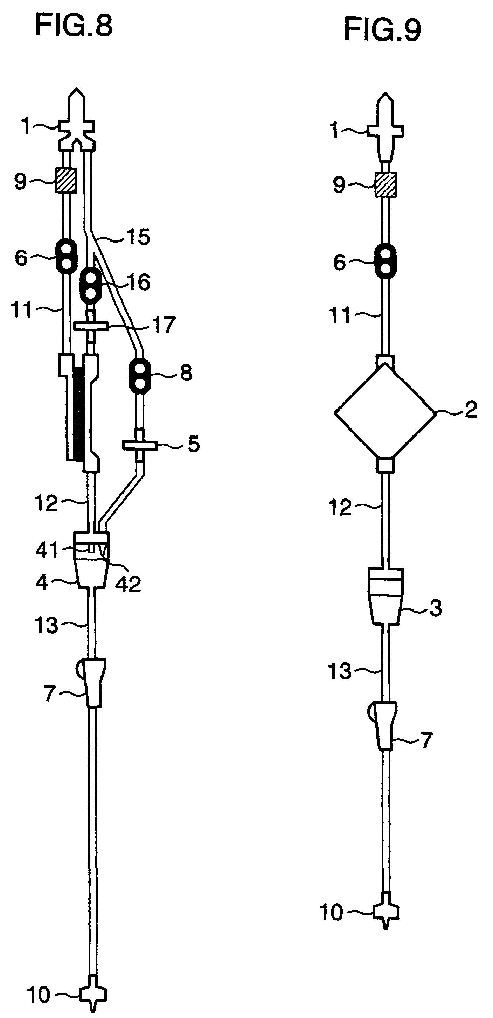

Fig. 8 is a schematic view showing still another embodiment of the present invention.

In the embodiment shown in Fig. 8, a blocking device (6), a blocking device (7) and a combination of blocking devices (8) and (16) are attached to a first conduit (11), a third conduit (13) and a bypass tube (15), respectively, and can be opened or shut as required. In addition, gas-liquid separation filters (5) and (17) are provided in the bypass tube (15) so that a blood product may stop when it reaches any of the gas-liquid separation filters, whereby non-filtered blood product is prevented from being incorporated into leukocyte-free blood product downstream to the filter apparatus, from the second flow path of the puncture needle (1) through the bypass tube.

The blocking devices are devices for blocking the passage of gas and liquid, such as clamps, roller clamps equipped with a flow regulator, and forceps, and are preferably attached to the first conduit (11), a second conduit (12), the third conduit (13) and the bypass tube (15), depending on purpose of use. The blocking device (7) is preferably a roller clamp equipped with a flow regulator for the purpose of adjusting the rate of transfusion into a patient. As the blocking devices (6), (8) and (16), any clamps can be used.

First, the blocking devices (6), (7), (8) and (16) are shut at a state at which sterile air has been sealed up inside the leukocyte-removing filter unit, and a puncture needle (1) is allowed to communicate with a blood container containing a blood product to be filtered, so as not to permit the passage of liquid either in or out.

A gravity head of the blood product is attained in the leukocyte-removing filter unit either by opening the blocking devices (6) and (7) to introduce a small volume of the blood product into the first conduit (11) through the first flow path of the puncture needle (1), and shutting the blocking device (7), or by attaching a first reservoir (9) to the first conduit (11), grasping the first reservoir (9) and returning the same to the original state to introduce a small volume of the blood product into the first conduit (11) through the first flow path of the puncture needle (1).

The first reservoir is a container having a space which permits reservation of gas and liquid. It is preferable to reserve the blood product in the first reservoir in a volume of one-third to one-half the capacity of the reservoir, and connect the reservoir to the first conduit between the puncture needle (1) and a leukocyte-removing filter apparatus (2) so as not to permit the passage of liquid either in or out, in order to prevent the introduction of air into the leukocyte-removing filter apparatus and avoid blocking. As the first reservoir (9), there are also reservoirs equipped with a filter for removing very small aggregates.

Then, the blocking device (8) is opened, upon which the blood product is transferred to the first conduit (11), the first reservoir (9), the leukocyte-removing filter apparatus (2), the second conduit (12), a drip chamber (4) and then the bypass tube (15), and at the same time, the sterile air sealed up inside the leukocyte-removing filter unit is transferred to the first conduit (11), the first reservoir (9), the leukocyte-removing filter apparatus (2), the second conduit (12), the drip chamber (4) and then the bypass tube (15), recovered into the blood container through the second flow path of the puncture needle (1) and reserved therein.

The blood product introduced into the leukocyte-removing filter unit is reserved in the cylinder of the drip chamber in a volume of about one-half the capacity of the drip chamber and flows into the flow path (42) and then the bypass tube (15). Leukocytes in the blood product are removed by the passage of the blood product through the leukocyte-removing filter apparatus (2).

In this case, the blocking device (8) is shut immediately before the blood product introduced into the leukocyte-removing filter unit reaches the bypass (15). Alternatively, the leukocyte-removing filter unit is designed to stop the blood product when the blood product reaches a gas-liquid separation filter (5) provided in the bypass tube (15), and after the stop, the blocking device (8) is shut.

A connecting adapter (10) is attached to the downstream (patient side) end of the third conduit (13), and the blocking device (7) (a roller clamp equipped with a flow regulator) is opened to introduce the blood product into the third conduit (13). When the blood product reaches the connecting adapter (10), the blocking device (7) is shut and a transfusion needle is attached to the connecting adapter (10) to complete preparation for transfusion.

The connecting adapter (10) is an adapter for connecting a transfusion needle used for carrying out blood transfusion. As the connecting adapter (10), conventional ones such as a Luer-Locke type connector, expansion U-bend, etc. are preferably used. It is also possible to connect a transfusion needle to the connecting adapter (10) previously.

The blocking device (7) is opened, the transfusion needle is filled with the blood product to the tip, the blocking device (7) is shut, and the transfusion needle is put into the vein of a patient. Then, the blocking device (7) is opened little by little and the transfusion rate is adjusted while counting drips from a nozzle for dripping in the drip chamber. Thus, the blood product freed of leukocytes is transfused into the patient.

After the whole of the blood product to be filtered in the blood container have flowed out into the leukocyte-removing filter unit, the sterile air reserved in the blood container is transferred through the first flow path of the puncture needle (1) to the first conduit (11), the first reservoir (9) and then the upstream space in the leukocyte-removing filter apparatus (2), whereby the residual blood product upstream to the leukocyte-removing filter apparatus in the leukocyte-removing filter unit can be transferred in the direction of the second conduit (12) to be recovered.

Further, the blocking device (16) is opened to transfer the sterile air reserved in the blood container, through the second flow path of the puncture needle (1) to the bypass tube (15) and then the down-stream space in the leukocyte-removing filter apparatus (2), whereby the residual blood product downstream to the leukocyte-removing filter apparatus in the leukocyte-removing filter unit can be transferred in the direction of the downstream (patient side) end of the third conduit (13) to the second conduit (12), the drip chamber (4) and then the third conduit (13) to be recovered.

The present invention is illustrated in further detail with reference to the following examples.

### Example 1

The components of the leukocyte-removing filter unit shown in Fig. 6 were produced in the following sizes, respectively, in practice.

As the puncture needle (1), that having a needle outside diameter of 5.6 mm, a sectional area of the opening (101a) of a first flow path (101) of 3.5 mm² and a sectional area of the opening (102a) of a second flow path (102) of 1.0 mm² was used. As the first conduit (11), a poly(vinyl chloride) tube having an inside diameter of 2.9 mm and a length of 200 mm was used. As the second conduit (12), a poly(vinyl chloride) tube having an inside diameter of 2.9 mm and a length of 200 mm was used. As the third conduit (13), a poly(vinyl chloride) tube having an inside diameter of 2.9 mm and a length of 1000 mm was used. As the bypass tube (15), a poly(vinyl chloride) tube having an inside diameter of 2.9 mm and a length of 200 mm was used. As the first reservoir (9), a cylindrical poly(vinyl chloride) container having an inside diameter ⌀ of 18 mm and a length (an inside dimension) of 70 mm was used. As the drip chamber (3), a cylindrical poly(vinyl chloride) container having an inside diameter ⌀ of 18 mm and a length (an inside dimension) of 60 mm was used. As the blocking devices (6) and (8), commercially available clamps were used. As the blocking device (7), a commercially available roller clamp equipped with a flow regulator was used. As the gas-liquid separation filter (5), a commercially available hydrophobic filter was used. As the connecting adapter (10), a commercially available Luer-Locke type connector was used. As the leukocyte-removing filter apparatus (2), commercially available Sepacel RZ200 (mfd. by Asahi Medical Co., Ltd.) was used, and a connection opening for connecting the bypass tube to the downstream space in the leukocyte-removing filter apparatus (2) was added thereto. Using the above components, the leukocyte-removing filter unit was assembled according to Fig. 6, inserted into a sterilization bag, and sterilized by ethylene oxide gas.

The blocking devices (6), (7) and (8) were shut at a state at which sterile air had been sealed up inside the sterilized leukocyte-removing filter unit shown in Fig. 6, and the puncture needle (1) was allowed to communicate with a blood container containing 300 ml of a blood product to be filtered, so as not to permit the passage of liquid either in or out.

The blood container and the leukocyte-removing filter unit were suspended on an irrigator stand (an instillation stand).

The blood product was introduced into the first conduit (11) through the first flow path of the puncture needle (1) by grasping the first reservoir (9) and returning the same to the original state, and was reserved in the first reservoir (9) in a volume of one-half (6 ml) the capacity of the first reservoir (9).

A gravity head was attained in the leukocyte-removing filter unit by the introduction of the blood product into the first conduit (11) and the first reservoir (9).

When the blocking device (8) was opened, the blood product was transferred to the first conduit (11), the first reservoir (9), the leukocyte-removing filter apparatus (2) and then the bypass tube (15), and at the same time, the sterile air sealed up inside the leukocyte-removing filter unit was transferred to the first conduit (11), the first reservoir (9), the leukocyte-removing filter apparatus (2) and then the bypass tube (15), recovered into the blood container through the second flow path of the puncture needle (1) and reserved therein.

After the blood product reached the gas-liquid filter (5) provided in the bypass tube (15) and stopped flowing, the blocking device (8) was shut.

Only the drip chamber (3) was turned upside down and the blocking device (7) (a roller clamp) was opened to introduce the blood product into the second conduit (12) and then the drip chamber (3). When the blood product was reserved in the drip chamber (3) in a volume of one-half (6 ml) the capacity of the drip chamber (3), the drip chamber was returned to its original state. After the third conduit (13) was filled with the blood product, the blocking device (7) was shut.

The blood product was filtered by the passage through the leukocyte-removing filter apparatus (2).

A commercially available transfusion needle was connected to the connecting adapter (10) and the filtered blood product was allowed to flow out of the leukocyte-removing filter unit at a rate of 5 ml/min by regulating the blocking device (7) (roller clamp). That is, the filtered blood product was allowed to assume a state in which it was transfused into a patient.

After the whole of the blood product to be filtered in the blood container had flowed out into the leukocyte-removing filter unit, the sterile air in the blood container was transferred through the first flow path of the puncture needle (1) to the first conduit (11), the first reservoir (9) and then the upstream space in the leukocyte-removing filter apparatus (2), whereby the residual blood product upstream to the leukocyte-removing filter apparatus (2) in the leukocyte-removing filter unit was allowed to flow down in the direction of the second conduit (12).

After the residual blood product upstream to the leukocyte-removing filter apparatus (2) was thus allowed to flow down to be recovered, the blocking device (8) was opened to allow the sterile air in the blood container to flow down through the second flow path of the puncture needle (1) to the bypass tube (15), the downstream space in the leukocyte-removing filter apparatus (2), the second conduit (12), the drip chamber (3) and then the third conduit (13), whereby the residual blood product downstream to the leukocyte-removing filter apparatus (2) in the leukocyte-removing filter unit was allowed to flow down in the direction of the downstream end of the third conduit (13) to be recovered.

When 300 ml of the blood product was treated by carrying out the above steps in the order in which they are described, the total volume of the residual blood product was 19.8 ml.

### Example 2

The components of the leukocyte-removing filter unit shown in Fig. 8 were produced in the following sizes, respectively, in practice.

As the puncture needle (1), that having a needle outside diameter of 5.6 mm, a sectional area of the opening (101a) of a first flow path (101) of 3.5 mm² and a sectional area of the opening (102a) of a second flow path (102) of 1.0 mm² was used. As the first conduit (11), a poly(vinyl chloride) tube having an inside diameter of 2.9 mm and a length of 200 mm was used. As the second conduit (12), a poly(vinyl chloride) tube having an inside diameter of 2.9 mm and a length of 200 mm was used. As the third conduit (13), a poly(vinyl chloride) tube having an inside diameter of 2.9 mm and a length of 1000 mm was used. As the bypass tube (15) extending from the second flow path of the puncture needle (1) to the drip chamber (4) and communicating with them, a poly(vinyl chloride) tube having an inside diameter of 2.9 mm and a length of 500 mm was used. As the branch of the bypass tube (15) put forth from the middle of the bypass tube (15) and communicating with the downstream space in the leukocyte-removing filter apparatus, a poly(vinyl chloride) tube having an inside diameter of 2.9 mm and a length of 200 mm was used. As the first reservoir (9), a cylindrical poly(vinyl chloride) container having an inside diameter ⌀ of 18 mm and a length (an inside dimension) of 70 mm was used. As the drip chamber (4), a cylindrical poly(vinyl chloride) container having an inside diameter ⌀ of 18 mm and a length (an inside dimension) of 60 mm was used. As the nozzle for dripping (41) in the space in the drip chamber, a thin tube having an outside diameter of 3.0 mm, an inside diameter of 1.8 mm and a length (from the top of the space in the drip chamber) of 12 mm was used. As the flow path (42) in the space in the drip chamber, a thin tube having an outside diameter of 3.0 mm, an inside diameter of 1.8 mm and a length (from the top of the space in the drip chamber) of 25 mm was used. As the blocking devices (6), (8) and (16), commercially available clamps were used. As the blocking device (7), a commercially available roller clamp equipped with a flow regulator was used. As the gas-liquid separation filters (5) and (17), commercially available hydrophobic filters were used. As the connecting adapter (10), a commercially available Luer-Locke type connector was used. As the leukocyte-removing filter apparatus (2), commercially available Sepacel RZ200 (mfd. by Asahi Medical Co., Ltd.) was used, and a connection opening for connecting the bypass tube to the downstream space in the leukocyte-removing filter apparatus (2) was added thereto. Using the above components, the leukocyte-removing filter unit was assembled according to Fig. 8, inserted into a sterilization bag, and sterilized by ethylene oxide gas.

The blocking devices (6), (7), (8) and (16) were shut at a state at which sterile air had been sealed up inside the sterilized leukocyte-removing filter unit shown in Fig. 8, and the puncture needle (1) was allowed to communicate with a blood container containing 300 ml of a blood product to be filtered, so as not to permit the passage of liquid either in or out.

The blood container and the leukocyte-removing filter unit were suspended on an irrigator stand (an instillation stand).

The blood product was introduced into the first conduit (11) through the first flow path of the puncture needle (1) by grasping the first reservoir (9) and returning the same to the original state, and was reserved in the first reservoir (9) in a volume of one-half (6 ml) the capacity of the first reservoir (9).

A gravity head was attained in the leukocyte-removing filter unit by the introduction of the blood product into the first conduit (11) and the first reservoir (9).

When the blocking device (8) was opened, the blood product was transferred to the first conduit (11), the first reservoir (9), the leukocyte-removing filter apparatus (2), the second conduit (12), the drip chamber (4) and then the bypass tube (15), and at the same time, the sterile air sealed up inside the leukocyte-removing filter unit was transferred to the first conduit (11), the first reservoir (9), the leukocyte-removing filter apparatus (2), the second conduit (12), the drip chamber (4) and then the bypass tube (15), recovered into the blood container through the second flow path of the puncture needle (1) and reserved therein.

The blood product introduced into the leukocyte-removing filter unit was reserved in the lower part of cylinder of the drip chamber (4) in a volume of about one-half (6 ml) the capacity of the drip chamber and flowed into the flow path (42) and then the bypass tube (15). After the blood product reached the gas-liquid separation filter (5) provided in the bypass tube (15) and stopped flowing, the blocking device (8) was shut.

The blood product was filtered by the passage through the leukocyte-removing filter apparatus (2).

A commercially available transfusion needle was connected to the connecting adapter (10) and the filtered blood product was allowed to flow out of the leukocyte-removing filter unit at a rate of 5 ml/min by regulating the blocking device (7) (roller clamp). That is, the filtered blood product was allowed to assume a state in which it was transfused into a patient.

After the whole of the blood product to be filtered in the blood container had flowed out into the leukocyte-removing filter unit, the sterile air in the blood container was transferred through the first flow path of the puncture needle (1) to the first conduit (11), the first reservoir (9) and then the upstream space in the leukocyte-removing filter apparatus (2), whereby the residual blood product upstream to the leukocyte-removing filter apparatus (2) in the leukocyte-removing filter unit was allowed to flow down in the direction of the second conduit (12).

After the residual blood product upstream to the leukocyte-removing filter apparatus (2) was thus allowed to flow down to be recovered, the blocking device (16) was opened to allow the sterile air in the blood container to flow down through the second flow path of the puncture needle (1) to the bypass tube (15), the downstream space in the leukocyte-removing filter apparatus (2), the second conduit (12), the drip chamber (4) and then the third conduit (13), whereby the residual blood product downstream to the leukocyte-removing filter apparatus (2) in the leukocyte-removing filter unit was allowed to flow down in the direction of the downstream end of the third conduit (13) to be recovered.

When 300 ml of the blood product was treated by carrying out the above steps in the order in which they are described, the total volume of the residual blood product was 20.4 ml.

### Comparative Example 1

In this comparative example, the leukocyte-removing filter unit shown in Fig. 9 was produced as an example of conventional leukocyte-removing filter unit not having such a bypass tube as used in the present invention and using a puncture needle having a single flow path.

As the puncture needle (1), a commercially available plastics spike having a sectional area of opening of 6.5 mm² was used. As the first conduit (11), a poly(vinyl chloride) tube having an inside diameter of 2.9 mm and a length of 200 mm was used. As the second conduit (12), a poly(vinyl chloride) tube having an inside diameter of 2.9 mm and a length of 200 mm was used. As the first reservoir (9), a cylindrical poly(vinyl chloride) container having an inside diameter ⌀ of 18 mm and a length (an inside dimension) of 70 mm was used. As the drip chamber (3), a cylindrical poly(vinyl chloride) container having an inside diameter ⌀ of 18 mm and a length (an inside dimension) of 60 mm was used. As the blocking device (6), a commercially available clamp was used. As the blocking device (7), a commercially available roller clamp equipped with a flow regulator was used. As the connecting adapter (10), a commercially available Luer-Locke type connector was used. As the leukocyte-removing filter apparatus (2), commercially available Sepacel RZ200 (mfd. by Asahi Medical Co., Ltd.) was used. Using the above components, the leukocyte-removing filter unit was assembled according to Fig. 9, inserted into a sterilization bag, and sterilized by ethylene oxide gas.

The blocking devices (6) and (7) of the sterilized leukocyte-removing filter unit shown in Fig. 9 were shut, and the puncture needle (1) was allowed to communicate with a blood container containing 300 ml of a blood product to be filtered, so as not to permit the passage of liquid either in or out.

The blood container and the leukocyte-removing filter unit were suspended on an irrigator stand (an instillation stand).

The blood product was introduced into the first conduit (11) through the first flow path of the puncture needle (1) by grasping the first reservoir (9) and returning the same to the original state, and was reserved in the first reservoir (9) in a volume of one-half (6 ml) the capacity of the first reservoir (9).

A gravity head was attained in the leukocyte-removing filter unit by the introduction of the blood product into the first conduit (11) and the first reservoir (9).

Then, the blocking device (6) and the blocking device (7) (roller clamp) were opened to transfer the blood product through the puncture needle (1) to the first conduit (11), the first reservoir (9) and then the leukocyte-removing filter apparatus (2).

Further, the blood product was allowed to flow down to the second conduit (12). In this case, only the drip chamber (3) was turned upside down. When the blood product was reserved in the drip chamber (3) in a volume of one-half (6 ml) the capacity of the drip chamber (3), the drip chamber was returned to its original state. After the third conduit (13) was filled with the blood product, the blocking device (7) was shut.

The blood product was filtered by the passage through the leukocyte-removing filter apparatus (2).

A commercially available transfusion needle was connected to the connecting adapter (10) and the filtered blood product was allowed to flow out of the leukocyte-removing filter unit at a rate of 5 ml/min by regulating the blocking device (7) (roller clamp). That is, the filtered blood product was allowed to assume a state in which it was transfused into a patient.

When the whole of the blood product to be filtered in the blood container flowed out into the leukocyte-removing filter unit, the blood product stopped flowing with a portion thereof remaining in the leukocyte-removing filter unit.

When 300 ml of the blood product was treated by carrying out the above steps in the order in which they are described, the total volume of the residual blood product was 58.8 ml.

### Example 3

The components of the leukocyte-removing filter unit shown in Fig. 7 were produced in the following sizes, respectively, in practice.

As the puncture needle (1), that having a needle outside diameter of 5.6 mm, a sectional area of the opening (101a) of a first flow path (101) of 3.5 mm² and a sectional area of the opening (102a) of a second flow path (102) of 1.0 mm² was used. As the first conduit (11), a poly(vinyl chloride) tube having an inside diameter of 2.9 mm and a length of 200 mm was used. As the second conduit (12), a poly(vinyl chloride) tube having an inside diameter of 2.9 mm and a length of 500 mm was used. As the bypass tube (15), a poly(vinyl chloride) tube having an inside diameter of 2.9 mm and a length of 200 mm was used. As the first reservoir (9), a cylindrical poly(vinyl chloride) container having an inside diameter ⌀ of 18 mm and a length (an inside dimension) of 70 mm was used. As the blocking devices (6), (8) and (19), commercially available clamps were used. As the gas-liquid separation filter (5), a commercially available hydrophobic filter was used. As the second reservoir (18), a commercially available blood bag was used. As the leukocyte-removing filter apparatus (2), commercially available Sepacel RZ200 (mfd. by Asahi Medical Co., Ltd.) was used, and a connection opening for connecting the bypass tube to the downstream space in the leukocyte-removing filter apparatus (2) was added thereto. Using the above components, the leukocyte-removing filter unit was assembled according to Fig. 7, inserted into a sterilization bag, and sterilized by ethylene oxide gas.

The blocking devices (6), (8) and (19) were shut at a state at which sterile air had been sealed up inside the sterilized leukocyte-removing filter unit shown in Fig. 7, and the puncture needle (1) was allowed to communicate with a blood container containing 300 ml of a blood product to be filtered, so as not to permit the passage of liquid either in or out.

The blood container and the leukocyte-removing filter unit were suspended on an irrigator stand (an instillation stand).

The blood product was introduced into the first conduit (11) through the first flow path of the puncture needle (1) by grasping the first reservoir (9) and returning the same to the original state, and was reserved in the first reservoir (9) in a volume of one-half (6 ml) the capacity of the first reservoir (9).

A gravity head was attained in the leukocyte-removing filter unit by the introduction of the blood product into the first conduit (11) and the first reservoir (9).

When the blocking device (8) was opened, the blood product was transferred to the first conduit (11), the first reservoir (9), the leukocyte-removing filter apparatus (2) and then the bypass tube (15), and at the same time, the sterile air sealed up inside the leukocyte-removing filter unit was transferred to the first conduit (11), the first reservoir (9), the leukocyte-removing filter apparatus (2) and then the bypass tube (15), recovered into the blood container through the second flow path of the puncture needle (1) and reserved therein.

After the blood product reached the gas-liquid filter (5) provided in the bypass tube (15) and stopped flowing, the blocking device (8) was shut.

The blocking device (19) was opened to allow the blood product to flow down to the second conduit (12) and then the second reservoir (18) (namely, the blood product was filtered).

The blood product was thus filtered by the passage through the leukocyte-removing filter apparatus (2).

After the whole of the blood product to be filtered in the blood container had flowed out into the leukocyte-removing filter unit, the sterile air in the blood container was transferred through the first flow path of the puncture needle (1) to the first conduit (11), the first reservoir (9) and then the upstream space in the leukocyte-removing filter apparatus (2), whereby the residual blood product upstream to the leukocyte-removing filter apparatus (2) in the leukocyte-removing filter unit was allowed to flow down in the direction of the second conduit (12).

After the residual blood product upstream to the leukocyte-removing filter apparatus (2) was thus allowed to flow down to be recovered, the blocking device (8) was opened to allow the sterile air in the blood container to flow down through the second flow path of the puncture needle (1) to the bypass tube (15), the downstream space in the leukocyte-removing filter apparatus (2), the second conduit (12) and then the second reservoir (18), whereby the residual blood product downstream to the leukocyte-removing filter apparatus (2) in the leukocyte-removing filter unit was allowed to flow down in the direction of the second reservoir (18) to be recovered.

When 300 ml of the blood product was treated by carrying out the above steps in the order in which they are described, the total volume of the residual blood product was 20.0 ml.

### Comparative Example 2

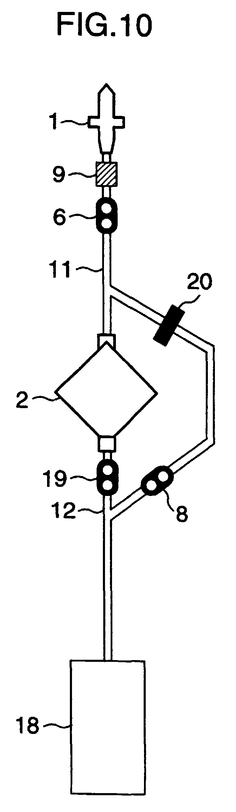

In this comparative example, the leukocyte-removing filter unit shown in Fig. 10 was produced as an example of conventional leukocyte-removing filter unit not having such a bypass tube as used in the present invention and using a puncture needle having a single flow path.

As the puncture needle (1), a commercially available plastics spike was used. As the first conduit (11), a poly(vinyl chloride) tube having an inside diameter of 2.9 mm and a length of 200 mm was used. As the second conduit (12), a poly(vinyl chloride) tube having an inside diameter of 2.9 mm and a length of 500 mm was used. As the bypass tube (15), a poly(vinyl chloride) tube having an inside diameter of 2.9 mm and a length of 400 mm was used. As the first reservoir (9), a cylindrical poly(vinyl chloride) container having an inside diameter ⌀ of 18 mm and a length (an inside dimension) of 70 mm was used. As the blocking device (6), (8) and (19), commercially available clamps were used. As the safety valve (20), a commercially available check valve permitting the passage of gas and liquid from the second conduit (12) side to the first conduit (11) side. As the second reservoir (18), a commercially available blood bag was used. As the leukocyte-removing filter apparatus (2), commercially available Sepacel RZ200 (mfd. by Asahi Medical Co., Ltd.) was used. Using the above components, the leukocyte-removing filter unit was assembled according to Fig. 10, inserted into a sterilization bag, and sterilized by ethylene oxide gas.

The blocking devices (6), (8) and (19) were shut at a state at which sterile air had been sealed up inside the sterilized leukocyte-removing filter unit shown in Fig. 10, and the puncture needle (1) was allowed to communicate with a blood container containing 300 ml of a blood product to be filtered, so as not to permit the passage of liquid either in or out.

The blood container and the leukocyte-removing filter unit were suspended on an irrigator stand (an instillation stand).

The blood product was introduced into the first conduit (11) through the first flow path of the puncture needle (1) by grasping the first reservoir (9) and returning the same to the original state, and was reserved in the first reservoir (9) in a volume of one-half (6 ml) the capacity of the first reservoir (9).

A gravity head was attained in the leukocyte-removing filter unit by the introduction of the blood product into the first conduit (11) and the first reservoir (9).

The blocking device (19) was opened to allow the blood product to flow down to the first conduit (11), the first reservoir (9), the leukocyte-removing filter apparatus (2), the second conduit (12) and then the second reservoir (18) (namely, the blood product was filtered), and at the same time, the sterile air sealed up inside the leukocyte-removing filter unit was transferred to the first conduit (11), the first reservoir (9), the leukocyte-removing filter apparatus (2), the second conduit (12) and then the second reservoir (18), recovered into the second reservoir (18) and reserved therein.

The blood product was filtered by the passage through the leukocyte-removing filter apparatus (2).

When the whole of the blood product to be filtered in the blood container flowed out into the leukocyte-removing filter unit, the blood product stopped flowing with a portion thereof remaining in the leukocyte-removing filter unit. The blocking device (19) was shut.

Then, the sterile air in the second reservoir (18) was returned upstream to the second conduit (12), the bypass tube (15), the first conduit (11) and the blood bag which had contained the blood product to be filtered, and the blocking device (8) was shut.

The blocking device (19) was opened, and the sterile air returned to the blood bag which had contained the blood product to be filtered was allowed to flow down to the first conduit (11), the first reservoir (9) and then the leukocyte-removing filter apparatus (2), and at the same time, the blood product remaining in the leukocyte-removing filter unit was transferred to the first conduit (11), the first reservoir (9) and then the upstream space in the leukocyte-removing filter apparatus (2) to be recovered. This residual blood product, however, stopped flowing with a portion thereof remaining in the portions of the filter unit which were downstream to the downstream space in the leukocyte-removing filter apparatus (2).

When 300 ml of the blood product was treated by carrying out the above steps in the order in which they are described, the total volume of the residual blood product was 30.8 ml.

### INDUSTRIAL APPLICABILITY

By the use of the leukocyte-removing filter unit of the present invention, blood or a blood component which remains in the leukocyte-removing filter unit can be recovered by simple operations by utilization of sterile air sealed up inside the leukocyte-removing filter unit, while maintaining sterility. The treated blood or blood component is wastefully discarded only in a greatly reduced amount, so that it has become possible to recover the precious blood or blood component in a larger amount than before. By the present invention, the leukocyte-removing filter unit requiring only easy operations and a low cost is provided.

## Claims

1. A leukocyte-removing filter unit comprising at least the following components a) to e):
a) a puncture needle (1) having therein a first flow path and a second flow path which are independent of each other,
b) a first conduit (11) connected to a down-stream end of the first flow path of said puncture needle,
c) a leukocyte-removing filter apparatus (2) comprising at least an upstream space connected to a down-stream end of said first conduit, a filter medium and a downstream space,
d) a second conduit (12) connected to the down-stream space of said leukocyte-removing filter apparatus, and
e) a bypass tube (15) one end of which is connected to the second flow path of said puncture needle (1) and the other end of which is connected to at least one of the downstream space in said leukocyte-removing filter apparatus (2) and said second conduit (12).

2. A leukocyte-removing filter unit comprising at least the following components a) to g):
a) a puncture needle (1) having therein a first flow path and a second flow path which are independent of each other,
b) a first conduit (11) connected to a down-stream end of the first flow path of said puncture needle,
c) a leukocyte-removing filter apparatus (2) comprising at least an upstream space connected to a down-stream end of said first conduit, a filter medium and a downstream space,
d) a second conduit (12) connected to the down-stream space of said leukocyte-removing filter apparatus,
e) a drip chamber (3) connected to a downstream end of said second conduit,
f) a third conduit (13) connected to a down-stream end of said drip chamber, and
g) a bypass tube (15) one end of which is connected to the second flow path of said puncture needle (1) and the other end of which is connected to at least one of the downstream space in said leukocyte-removing filter apparatus (2), said drip chamber (3) and said third conduit (13).

3. A leukocyte-removing filter unit comprising at least the following components a) to g):
a) a puncture needle (1) having therein a first flow path and a second flow path which are independent of each other,
b) a first conduit (11) connected to a down-stream end of the first flow path of said puncture needle,
c) a leukocyte-removing filter apparatus (2) comprising at least an upstream space connected to a down-stream end of said first conduit, a filter medium and a downstream space,
d) a second conduit (12) connected to the down-stream space of said leukocyte-removing filter apparatus,
e) a drip chamber (4) which is equipped with a nozzle (41) that communicates with an upper space in the drip chamber, and a flow path (42) one end of which opens outside said drip chamber and the other end of which opens downstream of said nozzle (41) in said drip chamber, said drip chamber (4) being connected to the downstream end of the second conduit (12) via said nozzle,
f) a third conduit (13) connected to a down-stream outlet of said drip chamber, and
g) a bypass tube (15) one end of which is connected to the second flow path of said puncture needle (1) and other ends of which are connected to the downstream space in said leukocyte-removing filter apparatus (2) and the flow path (42), respectively.

4. A leukocyte-removing filter unit according to any one of claims 1 to 3, which has a gas-liquid separation filter (5) provided therein so as to block said bypass tube (15).

5. A method for removing leukocytes from blood or a blood component by using a leukocyte-removing filter unit according to claim 1, which comprises carrying out at least the following steps a) to e) in this order:
a) placing a puncture needle (1) in communication with a blood container containing blood to be filtered, so as not to permit the passage of liquid either in or out,
b) causing the blood in said blood container to flow down through the first flow path of the puncture needle (1) to the first conduit (11), the leukocyte-removing filter apparatus (2) and then the second conduit (12),
c) causing sterile air inside the leukocyte-removing filter unit to flow into and remain in said blood container through the bypass tube (15) simultaneously with the above step b),
d) discharging blood remaining in the leukocyte-removing filter unit in the direction of the second conduit (12) by moving the sterile air in said blood container, through the first flow path of the puncture needle (1) to the first conduit (11) and then to the upstream space in the leukocyte-removing filter apparatus (2), after the whole of the blood in said blood container has flowed out, and/or
e) discharging blood remaining in the leukocyte-removing filter unit through the second conduit (12) by moving the sterile air in said blood container, through the second flow path of the puncture needle (1) and the bypass tube (15) to at least one of the downstream space in the leukocyte-removing filter apparatus (2) and the second conduit (12).

6. A method for removing leukocytes from blood or a blood component by using a leukocyte-removing filter unit according to claim 2, which comprises carrying out at least the following steps a) to e) in this order:
a) placing a puncture needle (1) in communication with a blood container containing blood to be filtered, so as not to permit the passage of liquid either in or out,
b) causing the blood in said blood container to flow down through the first flow path of the puncture needle (1) to the first conduit (11), the leukocyte-removing filter apparatus (2), the second conduit (12) and then the drip chamber (3),
c) causing sterile air inside the leukocyte-removing filter unit to flow into and remain in said blood container through the bypass tube (15) simultaneously with the above step b),
d) discharging blood remaining in the leukocyte-removing filter unit in the direction of the second conduit (12) by moving the sterile air in said blood container, through the first flow path of the puncture needle (1) to the first conduit (11) and then to the upstream space in the leukocyte-removing filter apparatus (2), after the whole of the blood in said blood container has flowed out, and/or
e) discharging blood remaining in the leukocyte-removing filter unit through the third conduit (13) by moving the sterile air in said blood container, through the second flow path of the puncture needle (1) and the bypass tube (15) to at least one of the downstream space in the leukocyte-removing filter apparatus (2), the drip chamber (3) and the third conduit (13).

7. A method for removing leukocytes from blood or a blood component by using a leukocyte-removing filter unit according to claim 3, which comprises carrying out at least the following steps a) to e) in this order:
a) placing a puncture needle (1) in communication with a blood container containing blood to be filtered, so as not to permit the passage of liquid either in or out,
b) causing the blood in said blood container to flow down through the first flow path of the puncture needle (1) to the first conduit (11), the leukocyte-removing filter apparatus (2), the second conduit (12) and then the drip chamber (4),
c) causing sterile air inside the leukocyte-removing filter unit to flow into and remain in said blood container through the bypass tube (15) simultaneously with the above step b),
d) discharging blood remaining in the leukocyte-removing filter unit in the direction of the second conduit (12) by moving the sterile air in said blood container, through the first flow path of the puncture needle (1) to the first conduit (11) and then to the upstream space in the leukocyte-removing filter apparatus (2), after the whole of the blood in said blood container has flowed out, and/or
e) discharging blood remaining in the leukocyte-removing filter unit through the third conduit (13) by moving the sterile air in said blood container, through the second flow path of the puncture needle (1) and the bypass tube (15) to the downstream space in the leukocyte-removing filter apparatus (2) and/or the drip chamber (4).

8. A puncture needle which has a first flow path (101) having an opening (101a) at one end on the puncture portion (103) and another opening (101b) at the other end, and a second flow path (102) having an opening (102a) at one end on the puncture portion (103) and another opening (102b) at the other end, wherein said first flow path and said second flow path are independent of each other, said opening (101a) is in a position lower than said opening (102a), and the cross-sectional area of said opening (101a) is larger than that of said opening (102a).
